# EUROPEAN PATENT APPLICATION

(11) **EP 3 466 927 A1**
(43) Date of publication of application: **10.04.2019**
(21) Application number: 17806527.2
(22) Date of filing: 25.05.2017
(51) Int. Cl.: C07D 213/16, C07D 213/22, C07D 213/61

(54) **METHOD FOR COUPLING OF HALOGENATED PYRIDINE COMPOUND WITH HALOGENATED AROMATIC COMPOUND**

(30) Priority: 30.05.2016 JP 2016107878
(71) Applicant: Kobelco Eco-Solutions Co., Ltd, Kobe-shi, Hyogo 651-0072 (JP)
(72) Inventor: MURAKAMI Yoshiaki, Kobe-shi Hyogo 651-2241 (JP); FUKUSHIMA Miyuki, Kobe-shi Hyogo 651-2241 (JP)
(74) Representative: Winter, Brandl, Fürniss, Hübner, Röss, Kaiser, Polte - Partnerschaft mbB
(86) International application number: PCT/JP2017/019595
(87) International publication number: WO 2017/208970

(57) **Abstract**

There is a demand for the development of a technique according to which a reaction for coupling a halogenated pyridine compound with a halogenated aromatic compound can be performed in a simple manner through a small number of steps without using expensive agents such as a palladium catalyst. A method for coupling a halogenated pyridine compound with a halogenated aromatic compound includes a step of coupling a halogenated pyridine compound with a halogenated aromatic compound to obtain a pyridine compound by reacting, in a reaction solvent, the halogenated pyridine compound and the halogenated aromatic compound with a solution containing an alkali metal.

## Description

### Technical Field

The present invention relates to a method for coupling a halogenated pyridine compound with a halogenated aromatic compound.

### Background Art

Pyridine compounds, particularly bipyridine compounds having, as a basic skeleton, a structure in which pyridine rings are linked to each other, phenylpyridine compounds having a structure in which a benzene ring is linked to a pyridine ring, and the like, are known to be useful as intermediates and raw materials of organic electroluminescent (organic EL) materials, pharmaceuticals, agricultural chemicals, and the like. These compounds are also known to be useful for a reaction for the reduction of carbon dioxide.

Ring structures such as pyridine rings and benzene rings are linked to each other through a coupling reaction to form a carbon-carbon bond between the ring structures. Various methods have been developed as such a coupling reaction, and coupling reactions performed using transition metal catalysts as typified by palladium are widely used. For example, the following technique is known as a method for manufacturing a disubstituted bipyridine compound obtained by dimerizing pyridine rings having one substituent: a disubstituted bipyridine compound is obtained by coupling halogenated pyridine compounds subjected to substitution using an alkyl group, a cyano group, a carboxyl group, or a group represented by -CO₂M (M represents an alkali metal) using a mixed solution of an aprotic solvent and water as a reaction solvent, in the presence of a palladium catalyst, a hydrazine, and a base such as an alkali metal hydroxide (see Patent Document 1, for example).

### Citation List

### Patent Literature

Patent Document 1: JP 2006-240997A

### Summary of Invention

### Technical Problem

However, a very expensive catalyst made of palladium or the like is used in a conventional coupling reaction, thus resulting in an increase in cost. In addition, it is necessary to carry out complicated steps for collection, regeneration, and the like of the catalyst in order to use the catalyst for an industrial application, thus resulting in an increase in the number of steps and complication of the process. In particular, palladium is a very rare noble metal, and therefore, there is also a problem regarding sustainability.

Therefore, there is a demand for the development of a technique according to which a reaction for coupling a halogenated pyridine compound with a halogenated aromatic compound can be performed in a simple manner through a small number of steps without using expensive agents such as a palladium catalyst.

### Solution to Problem

As a result of performing intensive studies to solve the foregoing problems, the inventors of the present invention found that a halogenated pyridine compound and a halogenated aromatic compound can be coupled with each other stably and efficiently without using a palladium catalyst or the like by reacting the halogenated pyridine compound and the halogenated aromatic compound with a dispersion product obtained by dispersing an alkali metal in a dispersion solvent or with a melt of an alkali metal. Such a coupling reaction does not require expensive agents such as a palladium catalyst and thus is economically advantageous. In addition, with such a coupling reaction, a halogenated pyridine compound and a halogenated aromatic compound can be coupled with each other in a simple manner in a short period of time through a small number of steps without requiring a complicated chemical technique. The inventors of the present invention achieved the present invention based on these findings.

That is, the present invention relates to a method for coupling a halogenated pyridine compound with a halogenated aromatic compound, and its feature is that the method includes a step of coupling a halogenated pyridine compound represented by General Formula (I) below with a halogenated aromatic compound represented by General Formula (II) below to obtain a pyridine compound represented by General Formula (III) below by reacting, in a reaction solvent, the halogenated pyridine compound and the halogenated aromatic compound with a solution containing an alkali metal; 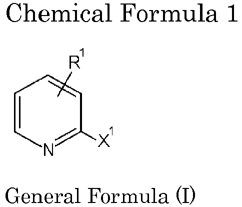 where X¹ in General Formula (I) represents a halogen atom and R¹ represents a hydrogen atom or a hydrocarbon group; 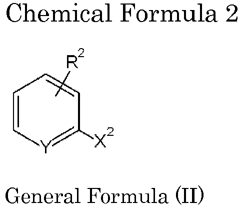 where X² in General Formula (II) represents a halogen atom and is optionally the same as or different from X¹ in General Formula (I), R² in General Formula (II) represents a hydrogen atom or a hydrocarbon group and is optionally the same as or different from R¹ in General Formula (I), and Y represents a carbon atom or a nitrogen atom; and 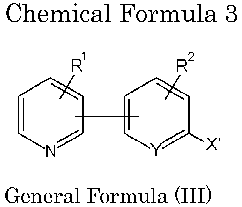 where X' in General Formula (III) represents a hydrogen atom or a halogen atom and is the same as X² in General Formula (II) in a case where X' represents a halogen atom, R¹ in General Formula (III) represents a hydrogen atom or a hydrocarbon group and is the same as R¹ in General Formula (I), R² in General Formula (III) represents a hydrogen atom or a hydrocarbon group and is the same as R² in General Formula (II), and Y in General Formula (III) represents a carbon atom or a nitrogen atom and is the same as Y in General Formula (II).

Conventionally, when a reaction for coupling a halogenated pyridine compound with a halogenated aromatic compound is performed, transition metal catalysts as typified by palladium are widely used. However, catalysts made of palladium or the like are very expensive, thus resulting in an increase in cost. In addition, it is necessary to provide steps for collection, regeneration, and the like of the catalyst in order to use the catalyst for an industrial application, thus resulting in an increase in the number of steps and complication of the process. With the above-mentioned configuration, a solution containing an alkali metal such as a dispersion product obtained by dispersing the alkali metal in a dispersion solvent or a melt of the alkali metal is used, and therefore, it is not necessary to use catalysts made of palladium or the like. Accordingly, it is possible to reduce the cost, and couple a halogenated pyridine compound with a halogenated aromatic compound in a simple manner in a short period of time through a small number of steps without requiring a complicated chemical technique. The configuration thus has significant economic and industrial advantages. In particular, alkali metals as typified by sodium are very widely distributed over the earth, and therefore, this technique is excellent from the viewpoint of sustainability. In addition, the coupling reaction can progress stably and efficiently.

Another feature is that the X¹ and the X² represent a chlorine atom, the R¹ and the R² represent a hydrogen atom, the Y represents a nitrogen atom, and the X' represents a hydrogen atom or a chlorine atom.

With this configuration, it is possible to provide a method for coupling 2-chloropyridine stably and efficiently without using a catalyst made of palladium or the like. As a result, 2,2'-bipyridine, 4,4'-bipyridine, and 6-chloro-2,2'-bipyridine that can be favorably used for manufacturing of organic EL materials, pharmaceuticals, agricultural chemicals, dyes, and the like, and a reaction for the reduction of carbon dioxide can be produced in a simple manner in a short period of time at low cost.

Another feature is that the reaction solvent includes chlorobenzene.

With this configuration, it is possible to produce 2,2'-bipyridine particularly efficiently and obtain highly pure 2,2'-bipyridine.

Another feature is that the X¹ represents a chlorine atom, the X² represents a bromine atom, the R¹ and the R² represent a hydrogen atom, the Y represents a carbon atom, and the X' represents a hydrogen atom.

With this configuration, it is possible to provide a method for coupling 2-chloropyridine with bromobenzene stably and efficiently without using a catalyst made of palladium or the like. As a result, 2-phenylpyridine that can be favorably used for manufacturing of organic EL materials, pharmaceuticals, agricultural chemicals, dyes, and the like, and a reaction for the reduction of carbon dioxide can be produced in a simple manner in a short period of time at low cost.

Another feature is that the X¹ and the X² represent a chlorine atom, the R¹ and the R² represent a methyl group, the Y represents a nitrogen atom, and the X' represents a hydrogen atom or a chlorine atom.

With this configuration, it is possible to provide a method for coupling 2-chloro-4-methylpyridine stably and efficiently without using a catalyst made of palladium or the like. As a result, 4,4'-dimethyl-2,2'-bipyridine, and 6-chloro-4,4'-dimethyl-2,2'-bipyridine that can be favorably used for manufacturing of organic EL materials, pharmaceuticals, agricultural chemicals, dyes, and the like, and a reaction for the reduction of carbon dioxide can be produced in a simple manner in a short period of time at low cost.

Another feature is that the reaction solvent is tetrahydrofuran.

With this configuration, using tetrahydrofuran (which may be abbreviated as "THF" hereinafter), which is an inexpensive solvent to be used for many purposes in the field of the art, makes it possible to provide a coupling method with more economic advantages. THF has excellent substance dissolvability, thus making it possible to improve the efficiency of contact between coupling target materials and a dispersion product obtained by dispersing an alkali metal in a dispersion solvent or a melt of an alkali metal, and thereby improve the efficiency of the coupling reaction. Accordingly, a highly pure coupling product can be obtained.

Another feature is that a usage amount of the solution containing an alkali metal is adjusted to obtain a predetermined halogenation ratio of the pyridine compound.

With this configuration, it is possible to control the halogenation ratio of the coupling product, namely a pyridine compound, thus making it possible to produce a pyridine compound depending on an application.

### Brief Description of Drawings

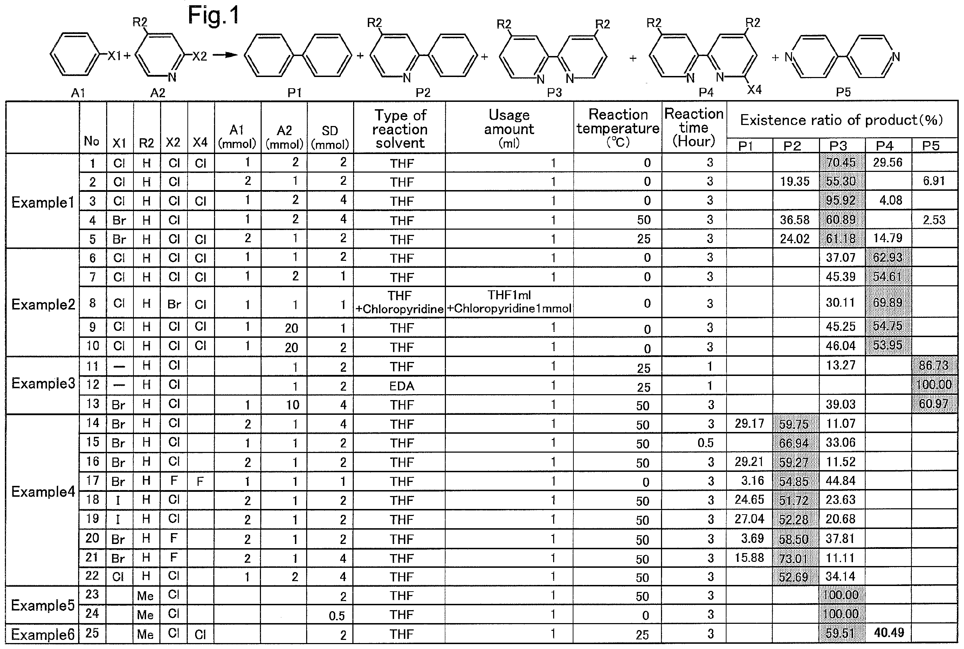

FIG. 1 is a diagram summarizing the coupling conditions and the coupling results in examples in which a method for coupling a halogenated pyridine compound with a halogenated aromatic compound according to an embodiment was investigated.

### Description of Embodiments

Hereinafter, a method for coupling a halogenated pyridine compound with a halogenated aromatic compound according to an embodiment of the present invention will be described in detail. However, the present invention is not limited to the embodiment described below.

With a coupling method according to this embodiment, a halogenated pyridine compound represented by General Formula (I) below and a halogenated aromatic compound represented by General Formula (II) below are coupled with each other by reacting, in a reaction solvent, the halogenated pyridine compound and the halogenated aromatic compound with a solution containing an alkali metal such as a dispersion product obtained by dispersing an alkali metal in a dispersion solvent or with a melt of an alkali metal, and a pyridine compound represented by General Formula (III) below is thus obtained. This coupling method requires no transition metal catalysts as typified by palladium. 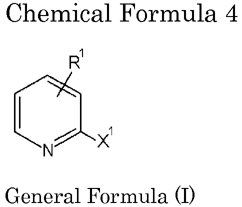 (Here, in General Formula (I), X¹ represents a halogen atom and R¹ represents a hydrogen atom or a hydrocarbon group.) 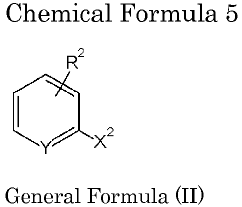 (Here, in General Formula (II), X² represents a halogen atom and is optionally the same as or different from X¹ in General Formula (I), R² represents a hydrogen atom or a hydrocarbon group and is optionally the same as or different from R¹ in General Formula (I), and Y represents a carbon atom or a nitrogen atom.) 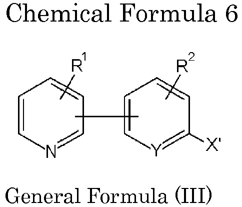 (Here, in General Formula (III), X' represents a hydrogen atom or a halogen atom and is the same as X² in General Formula (II) in a case where X' represents a halogen atom, R¹ represents a hydrogen atom or a hydrocarbon group and is the same as R¹ in General Formula (I), R² represents a hydrogen atom or a hydrocarbon group and is the same as R² in General Formula (II), and Y represents a carbon atom or a nitrogen atom and is the same as Y in General Formula (II).)

The coupling method according to this embodiment encompasses both homo-coupling with which two molecules having the same structure are linked to form one molecule and cross-coupling with which two molecules having different structures are linked to form one molecule.

In the halogenated pyridine compound represented by General Formula (I) serving as a coupling target compound of the coupling method according to this embodiment, a hydrogen atom at 2-position in the pyridine ring is substituted by a halogen atom. The halogen atom is selected from a fluorine atom (F), a chlorine atom (Cl), a bromine atom (Br), and an iodine atom (I).

In the halogenated pyridine compound represented by General Formula (I), a hydrogen atom at a position other than 2-position in the pyridine ring may also be substituted. For example, a hydrogen atom at such a position is substituted by a hydrocarbon group. There is no particular limitation on the hydrocarbon group as long as it contains preferably 1 to 10 carbon atoms and particularly preferably 1 to 5 carbon atoms. Therefore, the hydrocarbon group may be a linear, branched, or cyclic hydrocarbon group, and the hydrocarbon group may be a saturated or unsaturated hydrocarbon group. The hydrocarbon group is preferably a saturated linear or branched alkyl group, and specific examples thereof include a methyl group, an ethyl group, a propyl group, an isopropyl group, an n-butyl group, an isobutyl group, an s-butyl group, a t-butyl group, an n-pentyl group, an isopentyl group, a neopentyl group, a t-pentyl group, an s-pentyl group, a 2-methylbutyl group, a 1-ethylpropyl group, a 2-ethylpropyl group, an n-hexyl group, an isohexyl group, a neohexyl group, a t-hexyl group, a 2,2-dimethylbutyl group, a 2-methylpentyl group, a 3-methylpentyl group, a 1-ethylbutyl group, a 2-ethylbutyl group, a 1-propylpropyl group, an n-heptyl group, an isoheptyl group, an s-heptyl group, a t-heptyl group, a 2,2-dimethylpentyl group, a 3,3-dimethylpentyl group, a 1-methylhexyl group, a 2-methylhexyl group, a 3-methylhexyl group, a 4-methylhexyl group, a 1-ethylpentyl group, a 2-ethylpentyl group, a 3-ethylpentyl group, a 1-propylbutyl group, a 2-propylbutyl group, an n-octyl group, an isooctyl group, a t-octyl group, a neooctyl group, a 2,2-dimethylhexyl group, a 3,3-dimethylhexyl group, a 4,4-dimethylhexyl group, a 1-methylheptyl group, a 2-methylheptyl group, a 3-methylheptyl group, a 4-methylheptyl group, a 5-methylheptyl group, a 1-ethylhexyl group, a 2-ethylhexyl group, a 3-ethylhexyl group, a 4-ethylhexyl group, a 1-propylpentyl group, a 2-propylpentyl group, a 3-propylpentyl group, an n-nonyl group, an isononyl group, a t-nonyl group, a 1-methyloctyl group, a 2-methyloctyl group, a 3-methyloctyl group, a 4-methyloctyl group, a 5-methyloctyl group, a 6-methyloctyl group, an n-decyl group, an isodecyl group, a t-decyl group, a 1-methylnonyl group, a 2-methylnonyl group, a 3-methylnonyl group, a 4-methylnonyl group, a 5-methylnonyl group, a 6-methylnonyl group, and a 7-methylnonyl group.

A commercially available compound or a compound manufactured using a method known in the art may be used as the halogenated pyridine compound represented by General Formula (I).

In the halogenated aromatic compound represented by General Formula (II) serving as the other coupling target compound of the coupling method according to this embodiment, a single hydrogen atom at any position in the aromatic ring is substituted by a halogen atom. When the aromatic ring is a pyridine ring, a hydrogen atom at 2-position in the pyridine ring is substituted by a halogen atom. The halogen atom is selected from a fluorine atom (F), a chlorine atom (Cl), a bromine atom (Br), and an iodine atom (I). The halogen atom in the halogenated aromatic compound represented by General Formula (II) may be the same as or different from the halogen atom in the halogenated pyridine compound represented by General Formula (I).

In the halogenated aromatic compound represented by General Formula (II), a hydrogen atom at any position other than the position substituted by a halogen atom in the aromatic ring may also be substituted. For example, a hydrogen atom at such a position is substituted by a hydrocarbon group. There is no particular limitation on the hydrocarbon group as long as it contains preferably 1 to 10 carbon atoms and particularly preferably 1 to 5 carbon atoms. Specific examples of the hydrocarbon group include those described above for the halogenated pyridine compound represented by General Formula (I).

When the halogenated aromatic compound represented by General Formula (II) has been subjected to substitution using a hydrocarbon group, the hydrocarbon group may be the same as or different from the hydrocarbon group in the halogenated pyridine compound represented by General Formula (I). Moreover, only one or both of the halogenated pyridine compound represented by General Formula (I) and the halogenated aromatic compound represented by General Formula (II) may have been subjected to substitution using a hydrocarbon group.

A commercially available compound or a compound manufactured using a method known in the art may be used as the halogenated aromatic compound represented by General Formula (II).

Here, the "aromatic compound" collectively refers to compounds containing an aromatic ring, and the aromatic ring is defined as a ring structure that satisfies the Hückel rule, namely a conjugated unsaturated ring structure in which the number of electrons contained in the π-electron system on the ring is 4N+2 (N represents 0 or an integer). The aromatic compound according to this embodiment includes an aromatic hydrocarbon compound in which the skeleton of the ring structure is constituted by only carbon atoms and a heteroaromatic compound in which the skeleton of the ring structure contains an element other than carbon, and it is preferable that the element other than carbon is nitrogen. Specifically, an aromatic hydrocarbon compound containing a benzene ring or a heteroaromatic compound containing a pyridine ring is preferable.

According to the above-mentioned definition, the above-described halogenated pyridine compound is also a type of aromatic compound. In the coupling method according to this embodiment, at least one of the coupling target compounds is a halogenated pyridine compound, and therefore, in order to clarify this point, the coupling target compounds are expressed as a halogenated pyridine compound and a halogenated aromatic compound.

The following are favorable examples of a combination of the halogenated pyridine compound and the halogenated aromatic compound serving as the coupling target compounds of the coupling method according to this embodiment. However, there is no limitation to these combinations.
A. A combination of 2-chloropyridine, which is a halogenated pyridine compound represented by General Formula (I) in which X¹ represents a chlorine atom and R¹ represents a hydrogen atom, and 2-chloropyridine, which is a halogenated aromatic compound represented by General Formula (II) in which X² represents a chlorine atom, R² represents a hydrogen atom, and Y represents a nitrogen atom, that is, homo-coupling of 2-chloropyridine.
B. A combination of 2-chloropyridine, which is a halogenated pyridine compound represented by General Formula (I) in which X¹ represents a chlorine atom and R¹ represents a hydrogen atom, and bromobenzene, which is a halogenated aromatic compound represented by General Formula (II) in which X² represents a bromine atom, R² represents a hydrogen atom, and Y represents a carbon atom, that is, cross-coupling of 2-chloropyridine and bromobenzene.
C. A combination of 2-chloro-4-methylpyridine, which is a halogenated pyridine compound represented by General Formula (I) in which X¹ represents a chlorine atom and R¹ represents a methyl group, and 2-chloro-4-methylpyridine, which is a halogenated aromatic compound represented by General Formula (II) in which X² represents a chlorine atom, R² represents a methyl group, and Y represents a nitrogen atom, that is, homo-coupling of 2-chloro-4-methylpyridine.

An example of a solution containing an alkali metal used in the coupling method according to this embodiment is a dispersion product obtained by dispersing an alkali metal in a dispersion solvent. The dispersion product obtained by dispersing an alkali metal in a dispersion solvent is a dispersion product obtained by dispersing minute particles of an alkali metal in an insoluble solvent, or a dispersion product obtained by dispersing an alkali metal in a liquid form in an insoluble solvent. Examples of the alkali metal include sodium, potassium, lithium and alloys thereof. The average particle diameter of the minute particles is preferably less than 10 µm, and the minute particles having an average particle diameter of less than 5 µm can be used particularly preferably. The diameter of a sphere having a projected area equal to the projected area obtained through image analyses of photomicrographs is taken as the average particle diameter.

A solvent known in the art can be used as the dispersion solvent as long as minute particles of an alkali metal or an alkali metal in a liquid form can be dispersed in an insoluble solvent, and the reaction of the coupling target compounds with the dispersion product of the alkali metal is not inhibited. Examples thereof include aromatic solvents such as xylene and toluene, normal paraffin-based solvents such as decane, and mixed solvents thereof.

In the coupling method according to this embodiment, in addition to the dispersion product obtained by dispersing an alkali metal in a dispersion solvent, a melt obtained by melting an alkali metal can also be used in the same manner. A melt can be obtained by heating an alkali metal to its melting temperature using a means known in the art.

Hereinafter, the dispersion product obtained by dispersing an alkali metal in a dispersion solvent and a melt obtained by melting an alkali metal may be abbreviated as "SD". SD is an abbreviation of "sodium dispersion". In examples described below, a dispersion product in which sodium is dispersed as the alkali metal is used, and therefore, the dispersion product is denoted by the abbreviation "SD". However, alkali metals other than sodium are also encompassed by the abbreviation "SD", and the abbreviation "SD" is construed as encompassing not only a dispersion product but also a melt.

A solvent known in the art can be used as the reaction solvent used in the coupling method according to this embodiment as long as the reaction of the coupling target compounds with SD is not inhibited. Examples thereof include ether-based solvents, normal paraffin-based solvents, aromatic solvents, amine-based solvents, and heterocyclic compound-based solvents. As the ether-based solvent, a cyclic ether-based solvent is preferable, and tetrahydrofuran (which may be abbreviated as "THF" hereinafter) is particularly preferable. As the normal paraffin-based solvent, normal decane or the like is particularly preferable. As the aromatic solvent, xylene, toluene, benzene, or the like is preferable, and a halogenated aromatic solvent such as chlorobenzene or fluorobenzene can be used. As the amine-based solvent, ethylenediamine (which may be abbreviated as "EDA" hereinafter) or the like can be favorably used. As the heterocyclic compound-based solvent, tetrahydrothiophene or the like can be used. These solvents may be used alone or in combination of two or more as a mixed solvent. Here, the above-described dispersion solvent and the reaction solvent may be the same or different.

With the coupling method according to this embodiment, the halogenated pyridine compound represented by General Formula (I) and the halogenated aromatic compound represented by General Formula (II), which are coupling target compounds, are coupled with each other in the reaction solvent in the presence of SD. Hereinafter, this coupling reaction will be described in detail.

There is no particular limitation on the reaction temperature during the coupling reaction, and the reaction temperature can be set as appropriate depending on the types and amounts of the coupling target compounds, SD, and the reaction solvent, the reaction pressure, and the like. Specifically, it is preferable to set the reaction temperature to a temperature that is not higher than the boiling point of the reaction solvent. Under increased pressure, a boiling point is higher than that under atmospheric pressure, and the reaction temperature can thus be set to a higher temperature. The reaction can also be performed at room temperature, and the reaction temperature is preferably 0 to 100°C, and particularly preferably 20 to 80°C. Although it is not necessary to provide a particular temperature controlling means for heating, cooling, and the like, a temperature controlling means may be provided as necessary.

There is also no particular limitation on the reaction time, and it is sufficient that the reaction time is set as appropriate depending on the reaction temperature, the types and amounts of the coupling target compounds, SD, and the reaction solvent, the reaction pressure, and the like. In general, the reaction time is 1 to 24 hours and preferably 1 to 6 hours.

Moreover, all of the agents required in the coupling method according to this embodiment, namely the coupling target compounds, SD, the reaction solvent, and the like, can be handled stably in an atmosphere, thus making it possible to perform the reaction under normal pressure conditions in an atmosphere. However, the reaction may also be performed in an inert gas atmosphere that is filled with argon gas, nitrogen gas, or the like, or under increased pressure or reduced pressure, and there is no particular limitation.

Here, when the coupling target compounds are reacted with SD, it is preferable that 1 to 4 ml of the reaction solvent is used with respect to 1 mmol of the coupling target compounds, and SD is reacted with the coupling target compounds in the reaction solvent under conditions where the ratio of the amount of the coupling target compounds to the amount of SD is 1:1 to 1:3. In a case of cross-coupling, it is preferable to prepare the halogenated pyridine compound, the halogenated aromatic compound, and SD such that a molar equivalent ratio therebetween is 1:1:1 to 1:1:3. The substance amount of SD herein means the substance amount in terms of the alkali metal contained in SD.

The usage amount of SD can be set as appropriate depending on the types and amounts of the starting materials and the reaction solvent, but the usage amount of SD may affect the halogenation ratio of the coupling product. For example, when a small amount of SD is used, halogen derived from the coupling target compounds is likely to remain in the coupling product, whereas, if the usage amount of SD is increased, halogen derived from the coupling target compounds is not likely to remain in the coupling product, and the halogen atom is likely to be substituted by a hydrogen atom. Therefore, it is preferable to adjust the amount of SD depending on the desired coupling product.

With the coupling method according to this embodiment, the pyridine compound represented by General Formula (III) is obtained. In the pyridine compound, an aromatic ring is linked to a pyridine ring through a carbon-carbon bond. The pyridine ring and the aromatic ring in the pyridine compound represented by General Formula (III) may have been subjected to substitution using a hydrocarbon group, and the hydrocarbon group is derived from the halogenated pyridine compound represented by General Formula (I) and the halogenated aromatic compound represented by General Formula (II), which are coupling target compounds. Furthermore, the pyridine ring or aromatic ring in the pyridine compound represented by General Formula (III) may have been subjected to substitution using a halogen atom, and the halogen atom is also derived from the above-described coupling target compounds.

Specific examples of the pyridine compound represented by General Formula (III) obtained using the coupling method according to this embodiment include 2,2'-bipyridine, 4,4'-bipyridine, 6-chloro-2,2'-bipyridine, 2-phenylpyridine, 4,4'-dimethyl-2,2'-bipyridine, and 6-chloro-4,4'-dimethyl-2,2'-bipyridine.

The obtained pyridine compound represented by General Formula (III) may be purified using a purification means known in the art such as column chromatography, distillation, or recrystallization. Moreover, a configuration may be employed in which the residual unreacted coupling target compounds are collected and reused in the coupling reaction.

As described above, with the coupling method according to this embodiment, a single-step process progresses due to the addition of SD, and a halogenated pyridine compound and a halogenated aromatic compound can thus be coupled with each other stably and efficiently.

Conventionally, when a reaction for coupling a halogenated pyridine compound with a halogenated aromatic compound is performed, transition metal catalysts as typified by palladium are widely used. However, catalysts made of palladium or the like are very expensive, thus resulting in an increase in cost. In addition, it is necessary to provide steps for collection, regeneration, and the like of the catalyst in order to use the catalyst for an industrial application, thus resulting in an increase in the number of steps and complication of the process. With the above-mentioned configuration, a dispersion product obtained by dispersing an alkali metal in a dispersion solvent or a melt of an alkali metal is used, and therefore, it is not necessary to use catalysts made of palladium or the like. Accordingly, it is possible to reduce the cost, and couple a halogenated pyridine compound with a halogenated aromatic compound in a simple manner in a short period of time through a small number of steps without requiring a complicated chemical technique. The configuration thus has significant economic and industrial advantages. In particular, alkali metals as typified by sodium are very widely distributed over the earth, and therefore, this technique is excellent from the viewpoint of sustainability.

Furthermore, conventionally, a reaction for coupling two organic halogen compounds by reacting an alkali metal alone such as sodium metal with the two compounds is known as the Wurtz reaction. However, the Wurtz reaction is problematic in that there are limitations on coupling target compounds, and, in a case where an attempt is made to perform cross-coupling of different two organic halogen compounds, homo-coupling occurs preferentially, thus resulting in a significantly low yield of a target product, namely an aromatic compound obtained through cross-coupling. Since SD is used in the coupling method according to this embodiment, cross-coupling of a halogenated pyridine compound and a halogenated aromatic compound can be performed stably and efficiently. The coupling method according to this embodiment is advantageous in that the halogenation ratio of a coupling product, namely a pyridine compound, can be controlled depending on the addition amount of SD, and is different from the Wurtz reaction in this respect.

### Examples

Hereinafter, the present invention will be described in detail by use of examples, but the present invention is not limited to these examples. FIG. 1 summarizes the conditions and the results of the investigations in the following examples. In FIG. 1, A1 and A2 are starting materials, and P1 to P5 are products. The existence ratio of each product is a ratio to all the products shown as a percentage (%). It should be noted that a dispersion product obtained by dispersing minute particles of sodium metal in normal paraffin oil was used as SD in the following examples, and the substance amount of SD was a value in terms of sodium metal contained in SD.

### Example 1: Production of 2,2'-bipyridine through coupling of 2-chloropyridine

In 1 ml of THF, 2 mmol of 2-chloropyridine and 1 mmol of chlorobenzene were dissolved, and 2 mmol of SD was added thereto and reacted therewith at 0°C for 3 hours. The reaction was performed in an atmosphere. The results are shown in Experiment No. 1 in FIG. 1. As a result, as shown in Reaction Formula (1) below, 2,2'-bipyridine was obtained as a main product. Although not shown in Reaction Formula (1) below, 6-chloro-2,2'-bipyridine was produced as a by-product.

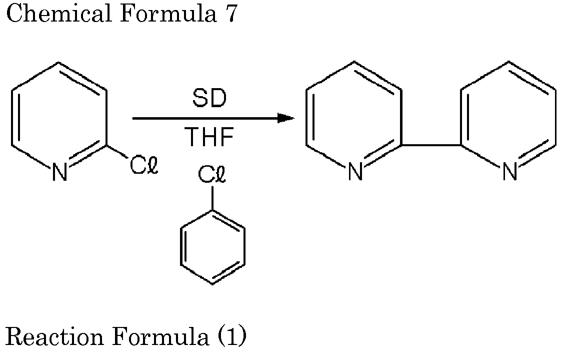

Moreover, 2,2'-bipyridine was obtained as a main product also in the cases where the reaction was performed under the conditions shown in Experiment Nos. 2 to 5 in FIG. 1.

### Example 2: Production of 6-chloro-2,2'-bipyridine through coupling of 2-chloropyridine

In 1 ml of THF, 2 mmol of 2-chloropyridine and 1 mmol of chlorobenzene were dissolved, and 1 mmol of SD was added thereto and reacted therewith at 0°C for 3 hours. The reaction was performed in an atmosphere. The results are shown in Experiment No. 7 in FIG. 1. As a result, as shown in Reaction Formula (2) below, 6-chloro-2,2'-bipyridine was obtained as a main product. Although not shown in Reaction Formula (2) below, 2,2'-bipyridine, which was a main product in Example 1, was produced as a by-product.

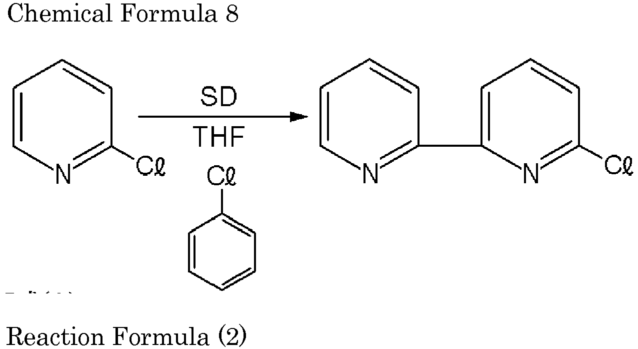

Moreover, 6-chloro-2,2'-bipyridine was obtained as a main product also in the cases where the reaction was performed under the conditions shown in Experiment Nos. 6 and 8 in FIG. 1. Furthermore, 6-chloro-2,2'-bipyridine was obtained as a main product also in the cases where the reaction was performed under the conditions shown in Experiment Nos. 9 and 10 in FIG. 1. Regarding Experiment Nos. 9 and 10, the amount of 2-chloropyridine with respect to SD was increased, but the production of 6-chloro-2,2'-bipyridine was not significantly affected.

It was revealed from the results of Examples 1 and 2 that, in the reaction system in which 2 mmol of 2-chloropyridine was used in 1 ml of THF, 2,2'-bipyridine was obtained as a main product when the molar equivalent ratio of 2-chloropyridine to SD was 1:1, and 6-chloro-2,2'-bipyridine, which is a halide, was obtained as a main product when the molar equivalent ratio of 2-chloropyridine to SD was 2:1 (comparison between Experiment Nos. 1 and 7 in FIG. 1).

### Example 3: Production of 4,4'-bipyridine through coupling of 2-chloropyridine

In 1 ml of THF, 1 mmol of 2-chloropyridine was dissolved, and 2 mmol of SD was added thereto and reacted therewith at 25°C for 1 hour. The reaction was performed in an atmosphere. The results are shown in Experiment No. 11 in FIG. 1. As a result, as shown in Reaction Formula (3) below, 4,4'-bipyridine was obtained as a main product. Although not shown in Reaction Formula (3) below, 2,2'-bipyridine was produced as a by-product.

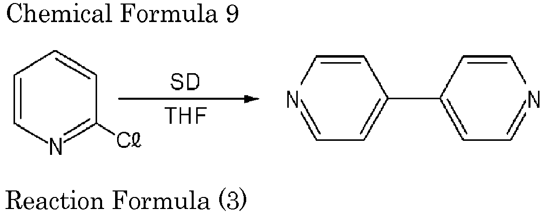

Moreover, 4,4'-bipyridine was obtained as a main product also in the cases where the reaction was performed under the conditions shown in Experiment Nos. 12 and 13 in FIG. 1.

It was revealed from the results of Examples 1, 2, and 3 that 2,2'-bipyridine was obtained as a main product when 2-chloropyridine was coupled in the presence of halogenated benzene such as chlorobenzene in 1 ml of THF under the conditions that 2 to 4 mmol of SD was added, and 6-chloro-2,2'-bipyridine, which is a halide, was obtained as a main product when the reaction was performed under the conditions that 1 to 2 mmol of SD was added (comparison between Experiment Nos. 1 to 5 and Experiment Nos. 6 to 10 in FIG. 1). Furthermore, it was revealed that 4,4-bipyridine was obtained as a main product when the reaction was performed in the absence of halogenated benzene or under the condition that a small amount of halogenated benzene had been added at a reaction temperature higher than or equal to 50°C (comparison between Experiment Nos. 1 to 10 and Experiment Nos. 11 to 13 in FIG. 1). Therefore, when the production of 4,4-bipyridine is desired, it is sufficient that the reaction is performed at a reaction temperature higher than or equal to 50°C under the condition that halogenated benzene is not added or a small amount of halogenated benzene is added, whereas when the prevention of the production of 4,4-bipyridine is desired, it is sufficient that the reaction is performed under the condition that the ratio of the addition amount of halogenated benzene to the addition amount of a pyridine compound is about 1:2 to 2:1 or the reaction temperature is low.

### Example 4: Production of 2-phenylpyridine through coupling of 2-chloropyridine and bromobenzene

In 1 ml of THF, 2-chloropyridine and bromobenzene in such amounts that the molar equivalent ratio of 2-chloropyridine to bromobenzene was 1:2 (1 mmol : 2 mmol) were dissolved, and 4 mmol of SD was added thereto and reacted therewith at 50°C for 3 hours. The reaction was performed in an atmosphere. The results are shown in Experiment No. 14 in FIG. 1. As a result, as shown in Reaction Formula (4) below, 2-phenylpyridine was obtained as a main product.

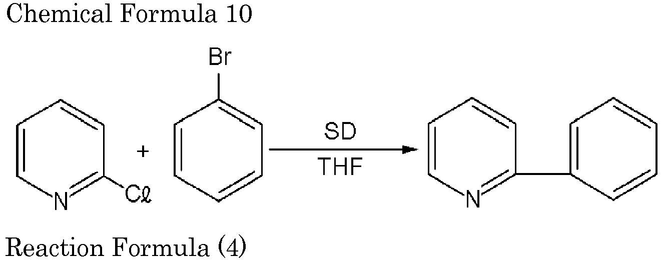

Moreover, 2-phenylpyridine was obtained as a main product also in the cases where the reaction was performed under the conditions shown in Experiment Nos. 15 to 22 in FIG. 1.

### Example 5: Production of 4,4'-dimethyl-2,2'-bipyridine through coupling of 2-chloro-4-methylpyridine

In 1 ml of THF, 2 mmol of 2-chloro-4-methylpyridine was dissolved, and 2 mmol of SD was added thereto and reacted therewith at 50°C for 3 hours. The reaction was performed in an atmosphere. The results are shown in Experiment No. 23 in FIG. 1. As a result, as shown in Reaction Formula (5) below, only 4,4'-dimethyl-2,2'-bipyridine was obtained as a product.

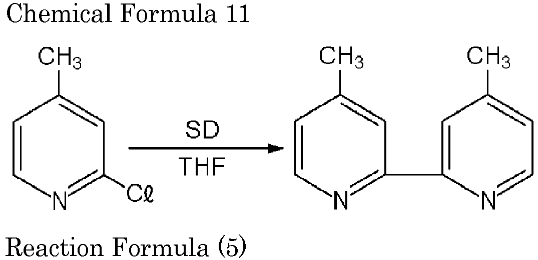

Moreover, only 2-phenylpyridine was obtained as a product also in the case where the reaction was performed under the conditions shown in Experiment No. 24 in FIG. 1.

### Example 6: Production of 6-chloro-4,4'-dimethyl-2,2'-bipyridine through coupling of 2-chloro-4-methylpyridine

In 1 ml of THF, 2 mmol of 2-chloro-4-methylpyridine was dissolved, and 2 mmol of SD was added thereto and reacted therewith at 25°C for 3 hours. The reaction was performed in an atmosphere. The results are shown in Experiment No. 25 in FIG. 1. As a result, as shown in Reaction Formula (6) below, 4,4'-dimethyl-2,2'-bipyridine was obtained as a main product. Although not shown in Reaction Formula (6) below, 6-chloro-4,4'-dimethyl-2,2'-bipyridine was produced as a by-product at a high ratio.

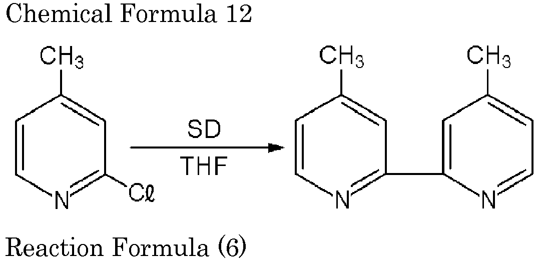

It was revealed from the results of Examples 5 and 6 that, in the reaction system in which 2 mmol of 2-chloro-4-methylpyridine was reacted with 2 mmol of SD in 1 ml of THF, 6-chloro-4,4'-dimethyl-2,2'-bipyridine was produced as a by-product by lowering the reaction temperature (comparison between Experiment Nos. 23 and 25). On the other hand, in the system in which the addition amount of SD was reduced to 0.5 mmol, even when the reaction temperature was lowered, only 4,4'-dimethyl-2,2'-bipyridine was produced, and the production of 6-chloro-4,4'-dimethyl-2,2'-bipyridine was not observed (Experiment No. 24 in FIG. 1). It was revealed from these results that the production of 4,4'-dimethyl-2,2'-bipyridine and 6-chloro-4,4'-dimethyl-2,2'-bipyridine, which is a halide of 4,4'-dimethyl-2,2'-bipyridine, can be controlled by changing the reaction temperature and the addition amount of SD as appropriate.

### Industrial Applicability

The present invention can be applied to a reaction for coupling a halogenated pyridine compound with a halogenated aromatic compound, and all the technical fields in which a pyridine compound obtained through such a coupling reaction is used, particularly manufacturing of organic EL materials, pharmaceuticals, agricultural chemicals, dyes, and the like and a reaction for the reduction of carbon dioxide.

## Claims

1. A method for coupling a halogenated pyridine compound with a halogenated aromatic compound, comprising a step of coupling a halogenated pyridine compound represented by General Formula (I) below with a halogenated aromatic compound represented by General Formula (II) below to obtain a pyridine compound represented by General Formula (III) below by reacting, in a reaction solvent, the halogenated pyridine compound and the halogenated aromatic compound with a solution containing an alkali metal: 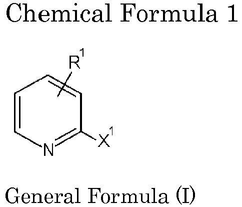 where X¹ in General Formula (I) represents a halogen atom and R¹ represents a hydrogen atom or a hydrocarbon group; 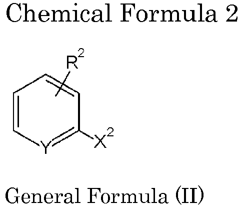 where X² in General Formula (II) represents a halogen atom and is optionally the same as or different from X¹ in General Formula (I), R² in General Formula (II) represents a hydrogen atom or a hydrocarbon group and is optionally the same as or different from R¹ in General Formula (I), and Y in General Formula (II) represents a carbon atom or a nitrogen atom; and 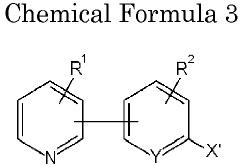 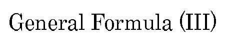 where X' in General Formula (III) represents a hydrogen atom or a halogen atom and is the same as X² in General Formula (II) in a case where X' represents a halogen atom, R¹ in General Formula (III) represents a hydrogen atom or a hydrocarbon group and is the same as R¹ in General Formula (I), R² in General Formula (III) represents a hydrogen atom or a hydrocarbon group and is the same as R² in General Formula (II), and Y in General Formula (III) represents a carbon atom or a nitrogen atom and is the same as Y in General Formula (II).

2. The method for coupling a halogenated pyridine compound with a halogenated aromatic compound according to claim 1, wherein the X¹ and the X² represent a chlorine atom, the R¹ and the R² represent a hydrogen atom, the Y represents a nitrogen atom, and the X' represents a hydrogen atom or a chlorine atom.

3. The method for coupling a halogenated pyridine compound with a halogenated aromatic compound according to claim 2, wherein the reaction solvent includes chlorobenzene.

4. The method for coupling a halogenated pyridine compound with a halogenated aromatic compound according to claim 1, wherein the X¹ represents a chlorine atom, the X² represents a bromine atom, the R¹ and the R² represent a hydrogen atom, the Y represents a carbon atom, and the X' represents a hydrogen atom.

5. The method for coupling a halogenated pyridine compound with a halogenated aromatic compound according to claim 1, wherein the X¹ and the X² represent a chlorine atom, the R¹ and the R² represent a methyl group, the Y represents a nitrogen atom, and the X' represents a hydrogen atom or a chlorine atom.

6. The method for coupling a halogenated pyridine compound with a halogenated aromatic compound according to any one of claims 1 to 5, wherein the reaction solvent is tetrahydrofuran.

7. The method for coupling a halogenated pyridine compound with a halogenated aromatic compound according to any one of claims 1 to 6, wherein a usage amount of the solution containing an alkali metal is adjusted to obtain a predetermined halogenation ratio of the pyridine compound.
